# EUROPEAN PATENT APPLICATION

(11) **EP 1 870 463 A1**
(43) Date of publication of application: **26.12.2007**
(21) Application number: 05727941.6
(22) Date of filing: 30.03.2005
(51) Int. Cl.: C12N 15/11

(54) **HIGH-AFFINITY RNA APTAMER MOLECULE FOR GLUTATHIONE S-TRANSFERASE PROTEIN**

(71) Applicant: Nec Soft, LTD., Tokyo 136-8627 (JP); National Institute of Advanced Industrial Science and Technology, Tokyo 100-8921 (JP)
(72) Inventor: YOSHIDA, Yoshihito, c/o NEC Soft, Ltd.,, Tokyo 1368627 (JP); PENMETCHA, Kumar, K.R., c/o National Institute, Tsukuba-shi (JP); NISHIKAWA, Satoshi, c/o National Institute, Tsukuba-shi (JP); WAGA, Iwao, c/o NEC Soft, Ltd.,, Tokyo 1368627 (JP)
(74) Representative: Glawe, Delfs, Moll
(86) International application number: PCT/JP2005/006130
(87) International publication number: WO 2006/103772

(57) **Abstract**

The present invention provides a "nucleic acid adaptor molecule" having specific binding affinity to a GST protein portion serving as an N-terminal fusion partner in a fusion protein consisting of the GST protein and a protein of interest. A "nucleic acid adaptor molecule against a GST protein" according to the present invention is an RNA aptamer molecule having any of the following nucleotide sequences I to III:

nucleotide sequence I (SEQ ID NO: 1): GGUAGAUACGAUGGA
UGGUUGUGUAAAGGUGGUCGUAUCCGCCGA CAUGACGCGCAGCCAA 61;

nucleotide sequence II (SEQ ID NO: 2): GGUAGAUACGAUGGA
CUAACUGCGCAAAUUACUCGUAUUAGCCGA CAUGACGCGCAGCCAA 61; or

nucleotide sequence III (SEQ ID NO: 3): GGUAGAUACGAUGGA
UACCGAAAAAUUAGUGUCGUUGACUGCAA CAUGACGCGCAGCCAA 60.

## Description

### Technical Field

The present invention relates to an RNA aptamer molecule exhibiting high affinity for a Glutathione-S-Transferase protein (GST; EC 2.5.1.18). Particularly, the present invention relates to an RNA aptamer molecule capable of binding with high affinity to a GST protein from Schistosoma japonicum, which has been utilized widely as a fusion partner in engineered production of fusion protein.

### Background Art

Glutathione-S-transferase proteins themselves have been known to be commonly present in a wide range of organisms from prokaryotes to eukaryotes. Their enzyme activity is such activity for catalyzing glutathione transfer reaction by using bond formation via a sulfanyl group (-SH) in the side chain of a cysteine residue contained in glutathione (N-(N-γ-L-glutamyl-L-cysteinyl)glycine). The enzyme activity itself of this GST protein is also important from the viewpoint of its involvement in detoxification processes via the binding of toxic substances to glutathione that is comparable to the binding thereof to glycine, glutamine, ornithine, cysteine, and the like, which is one of detoxification mechanisms.

On the other hand, the GST protein has been utilized as a fusion partner in the construction of fusion protein for the reasons that: the GST protein is capable of being recombinantly expressed as a soluble protein having its original steric structure and retaining enzyme activity in heterogenous hosts; and the GST protein is easily affinity column-purified by applying its property of specifically binding with high affinity to a substrate glutathione. For example, a GST protein from Schistosoma japonicum is utilized as a fusion partner in expression systems using E. coli as a host, leading to the expression of a fusion protein comprising a protein of interest linked via a linker sequence to the C-terminus of the GST protein. In such a system, the GST protein itself serving as an N-terminal fusion partner is recombinantly expressed as a soluble protein in the host E. coli, while the fusion protein is also expressed as a soluble protein in most cases. This feature is utilized for the purpose of obtaining a soluble protein in the form of a fusion protein even when a protein of interest itself forms an inclusion body in its recombinant expression in the host E. coli.

Since the GST protein portion serving as an N-terminal fusion partner has specific binding affinity to the substrate glutathione, affinity column purification to which this binding affinity is applied has been utilized for isolating and purifying the recombinantly expressed fusion protein. The fusion protein consisting of the GST protein and a protein of interest is initially bound onto a substrate glutathione-immobilized column, and contaminating proteins are eluted. Then, an eluent containing the substrate glutathione is supplied to the column to thereby dissolve the bond between the GST protein portion and the substrate glutathione immobilized on the column. Then, the fusion protein consisting of the GST protein and the protein of interest is collected. The purification means that utilizes the binding affinity between the substrate glutathione and the GST protein has high selectivity and has therefore been utilized in research on enzyme activity of a variety of proteins of interest because the fusion protein consisting of the GST protein and the protein of interest can be collected at high yields, for example, even from small amounts of cultures of recombinant expression products themselves.
On the other hand, there are some occasions where a single-stranded nucleic acid molecule of approximately 15 to 60 bases in length partially comprises an intra-molecular double-stranded structure attributed to base pairs between complementary bases (G-C, A-U, and A-T) and is folded in a three-dimensional structure as a whole, depending on its nucleotide sequence. Typical examples of natural single-stranded nucleic acid molecules being folded in a three-dimensional structure as a whole as a result of the partial formation of the intra-molecular double-stranded structure can include t-RNA molecules. There have been some reports suggesting that in the case of other various mRNA molecules, the partial formation of an intra-molecular double-stranded structure may also occur, and further they may be folded in a three-dimensional structure as a whole.

There has been reported such a phenomenon that even though a certain type of protein originally has no binding affinity to nucleic acids, a single-stranded nucleic acid molecule having a particular nucleotide sequence binds with high affinity onto the surface of the protein. Specifically, there are some cases of the single-stranded nucleic acid molecules having particular nucleotide sequences in which, when the single-stranded nucleic acid molecule having a particular nucleotide sequence causes at least the partial formation of an intra-molecular double-stranded structure occurs and thereby some three-dimensional structure as a whole is constructed, such a single-stranded nucleic acid molecule that constructs the three-dimensional structure may interact with plurality of sites on the surface of the three-dimensional structure which the certain type of protein has. When the single-stranded nucleic acid molecule that constructs the three-dimensional structure forms stable macro intermolecular bonds via the interaction thereof with the plurality of sites on the surface of the three-dimensional structure that the certain type of protein has, such type of single-stranded nucleic acid molecule is referred to as a "nucleic acid ligand against the protein" or a "nucleic acid adaptor molecule against the protein".

Of course, the three-dimensional structures themselves that proteins have include variety of steric structures. It is generally difficult to predict whether or not such a single-stranded nucleic acid molecule that constructs the three-dimensional structure, which is capable of forming stable macro intermolecular bonds, will be present for each of the variety of steric structures of proteins. Specifically, it is generally difficult to predict whether or not such a "specific antibody", which is capable of recognizing and binding to "three-dimensional epitope" that is formed from the steric structure of protein, will be present for each of proteins having variety of steric structures. In similar, it is generally difficult to predict whether or not such a "specific nucleic acid adaptor molecule having a three-dimensional structure", which recognizes "plurality of sites showing three-dimensional configuration" formed from the steric structure and forms stable macro intermolecular bonds therewith, will be present for each of proteins having variety of steric structures.

Thus, such an approach is used in which random screening-like technique is applied to experimentally confirm whether or not a "nucleic acid adaptor molecule against the protein" is actually present for individual protein. Specifically, in the approach used, a "random single-stranded nucleic acid molecule library" comprising single-stranded nucleic acid molecules in which a portion of a particular base length (N) having a random nucleotide sequence is inserted between 5'-terminal and 3'-terminal fixed regions is prepared as candidate single-stranded nucleic acid molecules and then subjected to actual screening to confirm whether or not a "single-stranded nucleic acid molecule" having binding affinity to the target protein is present.
For example, a screening approach for a "peptide" having binding affinity to a certain antibody by use of a phage-displayed "random peptide library" is based on the premise that an antigenic protein for the target "antibody" has a portion as an "epitope" for the "antibody", that is, a "particular amino acid sequence-comprising peptide fragment"-type "epitope sequence", in its peptide chain forming the protein. Specifically, this approach is based on the premise that the antigenic protein, not in a state of having its original three-dimensional structure but in a state of being denatured into a one-dimensional peptide chain, has an "epitope sequence" capable of binding to the target "antibody" through antigen-antibody reaction. If an "antibody" satisfies the premise, one peptide that has a "random peptide" portion having an amino acid sequence corresponding to the original "epitope sequence" for the target "antibody" is present with reliability in a phage-displayed "random peptide library" comprising peptides in which a "random peptide" portion of a particular amino acid length (N) having a random amino acid sequence is inserted between N-terminal and C-terminal fixed regions. Furthermore, a plurality (e.g. N×(20-1)) of peptides that have a "random peptide" portion having an amino acid sequence corresponding to a "one-amino acid substitution mutant" derived by one-amino acid substitution from the original "epitope sequence" for the target "antibody" are present with reliability in the library. In such a case, the peptide that has a "random peptide" portion having an amino acid sequence corresponding to the original "epitope sequence" exhibits the highest binding affinity to the target "antibody", while some of the plurality (e.g. N×(20-1)) of peptides that have a "random peptide" portion having an amino acid sequence corresponding to a "one-amino acid substitution mutant" exhibit considerably high binding affinity to the target "antibody".

In this approach using the phage-displayed "random peptide library", "random peptide-encoding genes" that encode peptide chains in which a "random peptide" portion of a particular amino acid length (N) having a random amino acid sequence is inserted between N-terminal and C-terminal fixed regions are first incorporated into phage vectors. E. coli hosts are infected with these "random peptide-encoding genes"-incorporated phage vectors, and cultured to cause the expression of the "random peptide" portion-inserted peptides chains. In a screening approach for an "epitope sequence" for the target "antibody", primary screening is conducted on the basis of the binding affinities of the "random peptide" portion-inserted peptide chains expressed in the E. coli hosts infected with the phage vectors to the target "antibody". A small "primary screening group" comprising the peptide that has a "random peptide" portion having an amino acid sequence corresponding to the original "epitope sequence" and the plurality of peptides that have a "random peptide" portion having an amino acid sequence corresponding to the "one-amino acid substitution mutant" is selected as E. coli hosts expressing the "random peptide" portion-inserted peptide chains bound with the target "antibody". The E. coli hosts contained in this "screening group" are cultured and subjected again to similar screening procedures. A "secondary screening group" thus obtained exhibits increases in the content of E. coli hosts expressing the peptide that has a "random peptide" portion having an amino acid sequence corresponding to the original "epitope sequence". Thus, a "panning" approach has been utilized in which with increases in the order of "screening", the resulting screening group efficiently achieves "exponential" increases in the content of E. coli hosts expressing the peptide that has a "random peptide" portion having an amino acid sequence corresponding to the original "epitope sequence".
There has been reported SELEX (Systematic Evolution of Ligands by Exponential Enrichment) (see U.S. Patent Nos. 5,475,096 and 5,270,163) that is a "random screening"-like approach applied to individual proteins to confirm whether or not a "nucleic acid adaptor molecule against the protein" is indeed present, which approach is corresponding to the "panning" approach utilized to select an "epitope sequence" for the target "antibody" from the phage-displayed "random peptide library".

In the SELEX method, a "random single-stranded nucleic acid molecule library" is constructed in such a fashion that a portion of a particular base length (N) having a random nucleotide sequence is inserted between 5'-terminal and 3'-terminal fixed regions. Then, a small "primary screening group" of "single-stranded nucleic acid molecules" exhibiting binding affinity to the target protein is selected. The "single-stranded nucleic acid molecules" contained in this "primary screening group" are used as templates to prepare cDNAs having nucleotide sequences complementary thereto. These cDNAs are further used as templates to perform PCR amplification using a PCR primer pair corresponding to the terminal portions of the 5'-terminal and 3'-terminal fixed regions. "Single-stranded nucleic acid molecules" used in secondary screening are prepared on the basis of the cDNAs contained in the resulting PCR amplification products. This group of "single-stranded nucleic acid molecules" for secondary screening is subjected again to similar screening procedures. A "secondary screening group" thus obtained exhibits increases in the content of "single-stranded nucleic acid molecules" being excellent in the binding affinity to the target protein. Thus, with increases in the order of "screening", the resulting screening group exhibits "exponential" increases in the content of "single-stranded nucleic acid molecules" being more excellent in the binding affinity to the target protein.

The multi-stage screening process in the SELEX method achieves "exponential" increases in the content of "single-stranded nucleic acid molecules" being more excellent in the binding affinity to the target protein. However, when a "nucleic acid adaptor molecule against the protein" exhibiting a certain level or higher of binding affinity to the target protein is originally absent, the "screening group" still contains considerable types of "single-stranded nucleic acid molecules" even after the completion of increased numbers of screening stages. In this regard, the SELEX method is essentially different from the "panning" approach, which can finally pick up the original "epitope sequence" for the target "antibody" with reliability. Specifically, whether or not a "single-stranded nucleic acid molecule" exhibiting a certain level or higher of the binding affinity to the target protein is originally present in the "random single-stranded nucleic acid molecule library" essentially depends on not "screening conditions" but the three-dimensional structure of the target protein.
Whether or not a "nucleic acid adaptor molecule against the protein" exhibiting a certain level or higher of binding affinity to the target protein is actually present depends just on the three-dimensional structure of the target protein. Moreover, such a "nucleic acid adaptor molecule against the protein" exhibiting a certain level or higher of the binding affinity is found with considerable probability to be absent for the target protein, depending on its three-dimensional structure. In this regard, this dependence is similar to dependence between a target protein and a "monoclonal antibody" such that the target protein is found with considerable probability to not effectively function as an immunogen and not induce the production of the monoclonal antibody, depending on its three-dimensional structure. If the actual presence of a "nucleic acid adaptor molecule against the protein" exhibiting a certain level or higher of binding affinity is confirmed by applying the SELEX method to a target protein, the obtained "nucleic acid adaptor molecule against the protein" is a "high-affinity nucleic acid adaptor molecule" specific to the target protein. This feature is similar to the relationship between a target protein having "immunogenicity" and its specific "monoclonal antibody".

### Disclosure of the Invention

### Problem to be Solved by the Invention

A variety of proteins of interest can be expressed recombinantly in the form of a fusion protein consisting of a GST protein and the protein of interest linked via a linker to the C-terminus of the GST protein serving as an N-terminal fusion partner, to thereby relatively easily prepare proteins of interest retaining their enzyme activity. This fusion protein consisting of the GST protein and the protein of interest can be subjected to affinity column purification and easily purified by using the specific binding affinity between the GST protein portion serving as an N-terminal fusion partner and a substrate glutathione. After this affinity column purification, as the substrate glutathione is in a state of being bound with the GST protein portion serving as an N-terminal fusion partner, the fusion protein consisting of the GST protein and the protein of interest eluted and collected must be subjected to final treatment for removing off the substrate glutathione therefrom.

Indeed, the affinity column purification method that utilizes the binding affinity to the substrate glutathione is a useful technique for purification of the fusion protein consisting of the GST protein and the protein of interest. However, some proteins of interest undergo denaturation under conditions of the treatment for removing the substrate glutathione from the GST protein. In this case, another affinity column purification method must be utilized instead of this approach to isolate the fusion protein consisting of the GST protein and the protein of interest. Specifically, it is desired to propose novel affinity column purification means that can serve as an alternative to the affinity column purification method utilizing the binding affinity to the substrate glutathione and has high selectivity for the GST protein portion serving as an N-terminal fusion partner.

If a "nucleic acid adaptor molecule against a GST protein" is available, this "nucleic acid adaptor molecule" can be used as a "ligand substrate having specific binding affinity to a GST protein" to thereby construct an affinity column purification method exhibiting high selectivity, which is comparable to that of antibody affinity column purification.

The present invention solves the problems, and an object of the present invention is to provide a "nucleic acid adaptor molecule" having specific binding affinity to a GST protein portion serving as an N-terminal fusion partner in a fusion protein consisting of the GST protein and a protein of interest. Another object of the present invention is to provide a novel affinity column purification method having high selectivity for the GST protein portion by applying, as a "nucleic acid ligand against the GST protein", the "nucleic acid adaptor molecule" having specific binding affinity to the GST protein portion serving as an N-terminal fusion partner.

### Means for Solving Problem

To attain the objects, the present inventor attempted to confirm whether or not a "nucleic acid adaptor molecule against the GST protein" exhibiting a certain level or higher of binding affinity to the GST protein from Schistosoma japonicum is indeed present. In the case if the "nucleic acid adaptor molecule against the GST protein" is present, the present inventor attempted to identify the nucleotide sequence of the single-stranded nucleic acid molecule composing the "nucleic acid adaptor molecule against the GST protein".

Specifically, a "random single-stranded nucleic acid molecule library" composed of single-stranded nucleic acid molecules, in which a portion of 30 bases in length (N₃₀) having a random nucleotide sequence was inserted between 5'-terminal and 3'-terminal fixed regions, was constructed. On the other hand, the GST protein from Schistosoma japonicum was recombinantly produced, and this recombinant GST protein was utilized to ascertain, by use of a SELEX method, whether or not a "nucleic acid adaptor molecule against the protein" exhibiting a certain level or higher of binding affinity thereto is actually present in the library.

As a result, in a screening process using the SELEX method, plurality of single-stranded RNA molecules exhibiting a certain level or higher of binding affinity to the recombinant GST protein were selected from among single-stranded RNA molecules transcribed from DNA molecules constituting the "random single-stranded nucleic acid molecule library". In addition, a fusion protein consisting of the GST protein and a protein of interest was utilized to conduct screening using the SELEX method in the same way. As a result, the plurality of single-stranded RNA molecules selected were proven to be single-stranded RNA molecules exhibiting a certain level or higher of binding affinity to the fusion protein consisting of the GST protein and the protein of interest. Hence, the plurality of single-stranded RNA molecules selected were verified to correspond to "nucleic acid adaptor molecules" having specific binding affinity to the GST protein portion serving as an N-terminal fusion partner in the fusion protein consisting of the GST protein and the protein of interest.

In addition to these findings, the present inventor analyzed the nucleotide sequences of the plurality of single-stranded RNA molecules selected, and completed the present invention on the basis of the results.

Specifically, "nucleic acid adaptor molecules against a GST protein" according to the present invention are single-stranded RNA molecules having the following three kinds of nucleotide sequences:
a "nucleic acid adaptor molecule against a GST protein" according to the first embodiment is
an RNA aptamer molecule capable of binding to a GST protein from Schistosoma japonicum, characterized in that
the RNA aptamer molecule is composed of
single-stranded RNA having the following nucleotide sequence I (SEQ ID NO: 1): 
GGUAGAUACGAUGGA UGGUUGUGUAAAGGUGGUCGUAUCCGCCGA
CAUGACGCGCAGCCAA 61;
a "nucleic acid adaptor molecule against a GST protein" according to the second embodiment is
an RNA aptamer molecule capable of binding to a GST protein from Schistosoma japonicum, characterized in that
the RNA aptamer molecule is composed of
single-stranded RNA having the following nucleotide sequence II (SEQ ID NO: 2): 
GGUAGAUACGAUGGA CUAACUGCGCAAAUUACUCGUAUUAGCCGA
CAUGACGCGCAGCCAA    61; and
a "nucleic acid adaptor molecule against a GST protein" according to the third embodiment is
an RNA aptamer molecule capable of binding to a GST protein from Schistosoma japonicum, characterized in that
the RNA aptamer molecule is composed
single-stranded RNA having the following nucleotide sequence III (SEQ ID NO: 3):

GGUAGAUACGAUGGA UACCGAAAAUUAGUGUCGUUGACUGCAA
CAUGACGCGCAGCCAA 60.

The present invention also provides the following two embodiments of methods for use of the "nucleic acid adaptor molecule against a GST protein" as applications of the "nucleic acid adaptor molecule against a GST protein":
a method for use of the "nucleic acid adaptor molecule against a GST protein" according to the first embodiment of the present invention is
use of any RNA aptamer molecule selected from the three kinds of RNA molecules mentioned above, characterized in that
the RNA aptamer molecule is used as a nucleic acid ligand substrate having specific binding affinity to a GST protein from Schistosoma japonicum, in preparation of an affinity column intended for affinity column purification of the GST protein or a fusion protein comprising the GST protein as a fusion partner and another protein linked to the C-terminus of the GST protein; and
a method for use of the "nucleic acid adaptor molecule against a GST protein" according to the second embodiment of the present invention is
use of any RNA aptamer molecule selected from the three kinds of RNA molecules mentioned above, characterized in that
the RNA aptamer molecule is used as a nucleic acid ligand substrate having specific binding affinity to a GST protein from Schistosoma japonicum, in preparation of a labeling substance intended for detection of a fusion protein comprising the GST protein as a fusion partner and another protein linked to the C-terminus of the GST protein.

### Effect of the Invention

The RNA aptamer molecule according to the present invention is a single-stranded RNA molecule exhibiting a specific binding affinity to the GST protein from Schistosoma japonicum and particularly exhibits a specific binding affinity to the GST protein portion serving as an N-terminal fusion partner in a fusion protein comprising the GST protein as an N-terminal fusion partner. In such a case, the binding of the RNA aptamer molecule onto the surface of the GST protein is achieved when this single-stranded RNA molecule is folded in a steric structure via the formation of an intra-molecular double-stranded structure attributed to its base pairs. On the other hand, the binding of the RNA aptamer molecule is dissolved when the double-stranded structure attributed to the base pairs is dissolved. Furthermore, the selection between the state in which the single-stranded RNA molecule takes up a steric structure via the formation of an intra-molecular double-stranded structure attributed to its base pairs and the state in which the double-stranded structure attributed to the base pairs is dissolved so that the single-stranded RNA molecule no longer exhibits the steric structure can be made reversibly by changing the concentration of a denaturant present in a liquid phase in which the single-stranded RNA molecule is placed. Thus, said RNA aptamer is characterized in that its binding affinity to the GST protein can be changed reversibly depending on changes in the structure of the single-stranded RNA molecule, and can be utilized as a nucleic acid ligand substrate having specific binding affinity to the GST protein in affinity column purification or as a nucleic acid ligand substrate having specific binding affinity to the GST protein in the preparation of a labeling substance intended for detection of a fusion protein comprising the GST protein as an N-terminal fusion partner.

### Brief Description of the Drawings

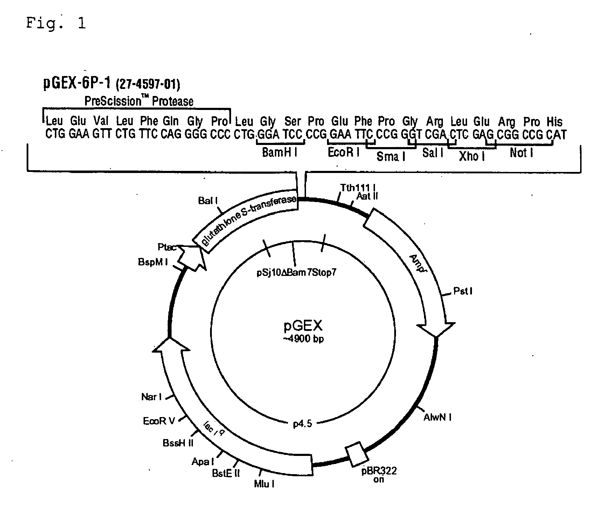

Figure 1 is a drawing showing the structure of a commercially available plasmid vector pGEX 6p-2 for GST protein expression utilized in the recombinant expression of a GST protein from Schistosoma japonicum and in the recombinant expression of a fusion protein consisting of the GST protein and a protein of interest;

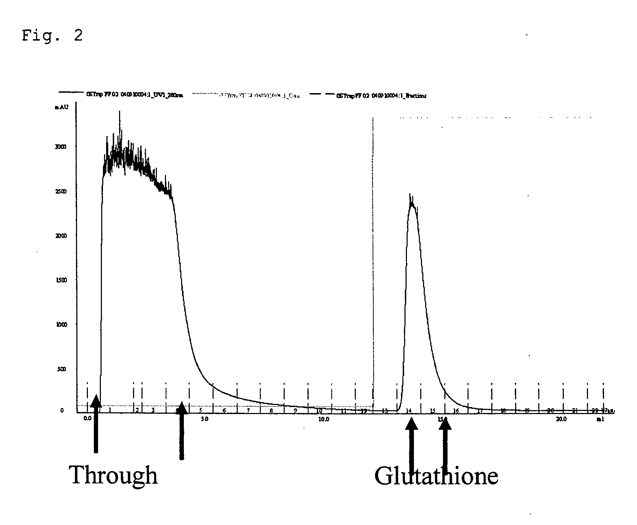

Figure 2 is a drawing showing the elution of a GST protein using glutathione in an elution curve from an affinity column GSTrap FF at a purification step of the GST protein recombinantly expressed in host E. coli by use of the GSTrap FF column;

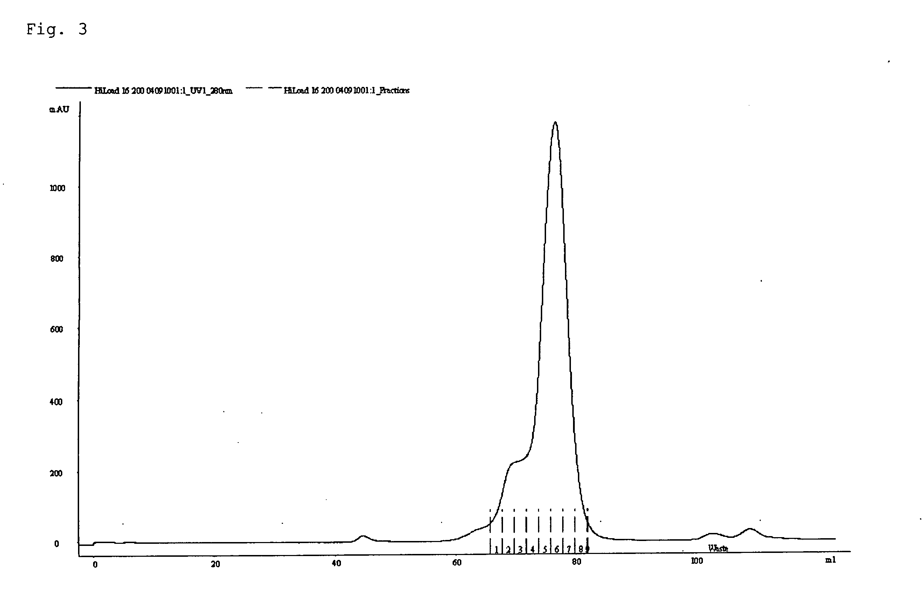

Figure 3 is a drawing showing a GST protein-containing elution fraction in an elution curve from a gel filtration column at a purification step by means of gel filtration with the HiLoad 16/10 200 pg after the purification using the GSTrap FF column;

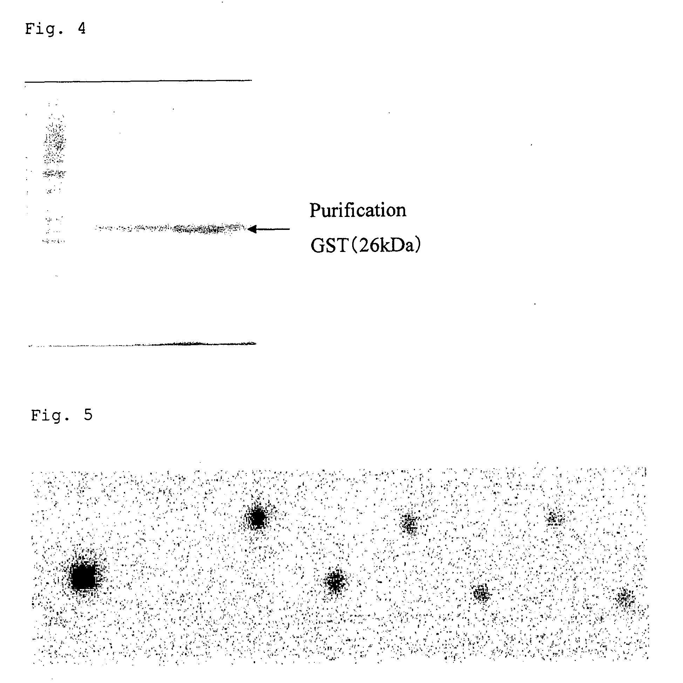

Figure 4 is a drawing showing a result of conducting SDS-PAGE analysis on the GST protein-containing elution fraction collected at the step of purification using the gel filtration column;

Figure 5 is a drawing showing a result of examining a group of single-stranded RNA molecules (the 18^{th} RNA pool) having a high and specific binding affinity to the GST protein, which was finally collected by a series of screening processes using a SELEX method, for their binding affinity to the GST protein by Filter Binding Assay;

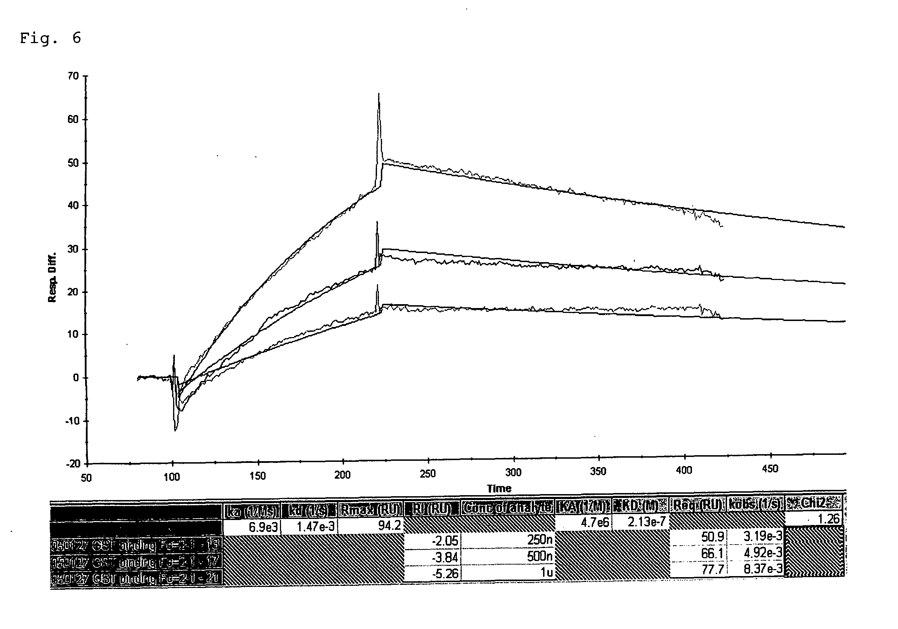

Figure 6 is a drawing showing a result of evaluating a single-stranded RNA molecule (No. 3 clone) having a high and specific binding affinity to the GST protein, which was selected by a series of screening processes using a SELEX method, for the process of formation of its complex with the GST protein and the process of dissociation of the complex by use of a surface plasmon resonance detection apparatus;

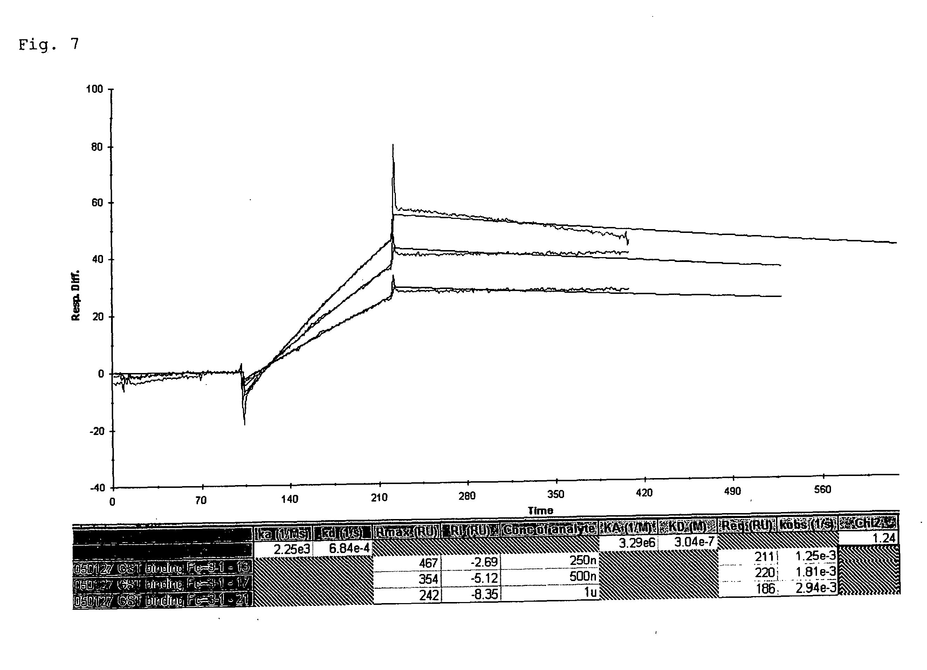

Figure 7 is a drawing showing a result of evaluating a single-stranded RNA molecule (No. 5 clone) having a high and specific binding affinity to the GST protein, which was selected by a series of screening processes using a SELEX method, for the process of formation of its complex with the GST protein and the process of dissociation of the complex by use of a surface plasmon resonance detection apparatus; and

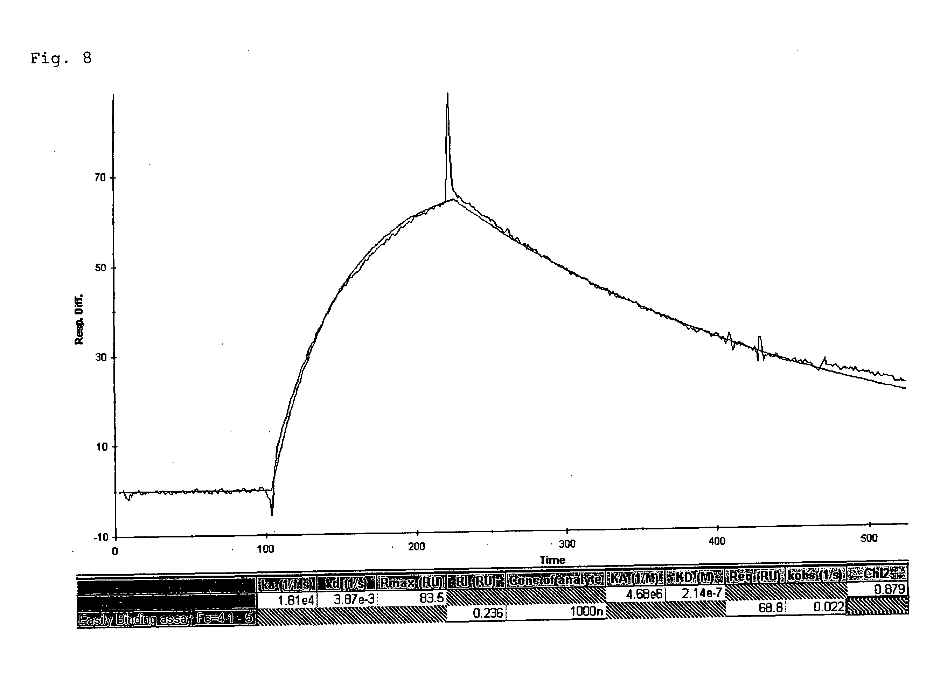

Figure 8 is a drawing showing a result of evaluating a single-stranded RNA molecule (No. 26 clone) having a high and specific binding affinity to the GST protein, which was selected by a series of screening processes using a SELEX method, for the process of formation of its complex with the GST protein and the process of dissociation of the complex by use of a surface plasmon resonance detection apparatus.

### Best Mode for Carrying Out the Invention

A "nucleic acid adaptor molecule against the GST protein" according to the present invention will be described more specifically below.

The "nucleic acid adaptor molecule against the GST protein" according to the present invention is a single-stranded RNA molecule exhibiting a high and specific binding affinity to the surface of the GST protein from Schistosoma japonicum or a fusion protein of the GST protein / a protein of interest type, which comprises the GST protein as an N-terminal fusion partner and the protein of interest linked to the C-terminus thereof. Specifically, the nucleic acid adaptor molecule was actually selected as a candidate single-stranded RNA molecule by use of a SELEX method from an RNA pool comprising single-stranded RNA molecules having the following nucleotide sequence R_{candidate0}:
5'-GGUAGAUACGAUGGA
   NNNNNNNNNN NNNNNNNNNN NNNNNNNNNN
   CAUGACGCGCAGCCAA-3'
   and having both terminal fixed regions and a "random nucleotide sequence region" of 30 bases in length inserted therebetween.

Specifically, the nucleic acid adaptor molecule was finally picked out as a single-stranded RNA molecule exhibiting an excellent binding affinity to the GST protein, as a result of a process, as explained in Example below, wherein
first, 13 rounds of SELEX selection using a Filter separation method are repeated;
additional 2 rounds of SELEX selection using a surface plasmon resonance biosensor are performed; and
finally, 2 rounds of SELEX selection using GST affinity beads are performed, whereby
with increases in the number of selection rounds, single-stranded RNA molecules having a poor binding affinity to the GST protein are removed in stages from the initial RNA pool comprising single-stranded RNA molecules having both terminal fixed regions and a "random nucleotide sequence region" of 30 bases in length inserted therebetween, whereas the content of those exhibiting an excellent binding affinity to the GST protein is increased in stages.

The RNA aptamer molecule according to the present invention can be prepared by in vitro transcription using any one of double-stranded DNA molecules described below as a transcription template with T7 RNA polymerase.

Specifically, a single-stranded RNA molecule having the following nucleotide sequence I (SEQ ID NO: 1): 
GGUAGAUACGAUGGA UGGUUGUGUAAAGGUGGUCGUAUCCGCCGA
CAUGACGCGCAGCCAA 61
 is prepared by use of a "T7 promoter region" (TGTAATACGACTCACTATA) from a transcription template having the following nucleotide sequence (SEQ ID NO: 4): 
TGTAATACGACTCACTATA GGTAGATACGATGGA
TGGTTGTGTAAAGGTGGTCGTATCCGCCGA CATGACGCGCAGCCAA
 which is inserted in a plasmid vector "pCR-GST No. 3" carried in No. 3 clone.

A single-stranded RNA molecule having the following nucleotide sequence II (SEQ ID NO: 2): 
GGUAGAUACGAUGGA CUAACUGCGCAAAUUACUCGUAUUAGCCGA
CAUGACGCGCAGCCAA 61
 is prepared by use of a "T7 promoter region" (TGTAATACGACTCACTATA) from a transcription template having the following nucleotide sequence (SEQ ID NO: 5): 
TGTAATACGACTCACTATA GGTAGATACGATGGA
CTAACTGCGCAAATTACTCGTATTAGCCGA CATGACGCGCAGCCAA
 which is inserted in a plasmid vector "pCR-GST No. 5" carried in No. 5 clone.

A single-stranded RNA molecule having the following nucleotide sequence III (SEQ ID NO: 3): 
GGUAGAUACGAUGGA UACCGAAAAUUAGUGUCGUUGACUGCAA
CAUGACGCGCAGCCAA 60
 is prepared by use of a "T7 promoter region" (TGTAATACGACTCACTATA) from a transcription template having the following nucleotide sequence (SEQ ID NO: 6): 
TGTAATACGACTCACTATA GGTAGATACGATGGA
TACCGAAAAATTAGTGTCGTTGACTGCAA CATGACGCGCAGCCAA
 which is inserted in a plasmid vector "pCR-GST No. 26" carried in No. 26 clone.
The plasmids carried in these 3 kinds of clones, No. 3, No. 5, and No. 26 clones, have been deposited domestically since Mar. 7, 2005 as deposition Nos. FERM P-20439 (designated as "pCR-GST No. 3" as indication for identification), FERM P-20440 (designated as "pCR-GST No. 5"), and FERM P-20441 (designated as "pCR-GST No. 26"), respectively, with International Patent Organism Depositary, National Institute of Advanced Industrial Science and Technology (Tsukuba Central 6, 1-1 Higashi, Tsukuba, Ibaraki 305-8566, Japan).
The "T7 promoter region" (TGTAATACGACTCACTATA) in the nucleotide sequences of the transcription templates is utilized in the in vitro transcription using T7 RNA polymerase and may be replaced by other nucleotide sequences that can be used as promoter sequences for the T7 RNA polymerase. Further, for example, a polyA sequence (Aₙ) may be inserted between the "T7 promoter region" (TGTAATACGACTCACTATA) and the 5'-terminal fixed region "GGTAGATACGATGGA" to prepare a single-stranded RNA molecule in which this polyA sequence (An) is added to the 5'-terminus thereof. In addition, the RNA adaptor molecule according to the present invention is a single-stranded RNA molecule of 100 bases or less, in which an additional nucleotide sequence such as the polyA sequence (An) is included, and thus the RNA adaptor molecule may be prepared altogether by chemical synthesis.
Alternatively, the RNA adaptor molecule according to the present invention may be prepared in such a form that the constituent bases are subject to such modification as 2'-fluoro(2'-F), 2'-amino(2'-NH₂) or 2'-O-methyl(2'-OCH₃) substitution, while retaining its nucleotide sequence. In addition, the principal chain constituting the single-stranded RNA molecule may be subject to such modification as 5'- and 3'-phosphorothioate capping or 3'-3' reverse phosphodiester linkage at the 3'-terminus thereof. These modifications have a function of imparting resistance to the degradation of the RNA molecule by RNase and have an effect of improving the stability of the single-stranded RNA molecule.
The RNA adaptor molecule according to the present invention exhibits specific and high binding affinity not only to the GST protein but also to the GST protein portion in a fusion protein comprising the GST protein as an N-terminal fusion partner. Because of this advantage, the RNA adaptor molecule can be utilized as a nucleic acid ligand substrate having specific binding affinity to the GST protein in affinity column purification or as a nucleic acid ligand substrate having specific binding affinity to the GST protein in the preparation of a labeling substance intended for detection of a fusion protein comprising the GST protein as an N-terminal fusion partner.

### Example

Hereinafter, a series of processes for selecting an "RNA adaptor molecule against a GST protein" according to the present invention by use of a SELEX method will be described specifically.

### "Random single-stranded nucleic acid molecule library"

In the process for selecting an "RNA adaptor molecule against a GST protein" by use of a SELEX method, a pool of single-stranded RNA molecules, which are transcribed from a "random DNA molecule library" having a nucleotide sequence described below, are used as a "random single-stranded nucleic acid molecule library" to be screened.

This "random DNA molecule library" comprises DNA molecules incorporating therein a random nucleotide sequence region, and the nucleotide sequence of each DNA molecule is represented by the following nucleotide sequence D_{randum0}:
5'-TGTAATACGACTCACTATA GGTAGATACGATGGA
   NNNNNNNNNN NNNNNNNNNN NNNNNNNNNN
   CATGACGCGCAGCCAA-3'
   and composed of the following 3 regions:
   the 5'-terminal fixed region: TGTAATACGACTCACTATA GGTAGATACGATGGA;
   the random region of 30 bases: N₃₀; and
   the 3'-terminal fixed region: CATGACGCGCAGCCAA.

TGTAATACGACTCACTATA included in the 5'-terminal fixed region is a "T7 promoter region" that serves as a promoter region in transcription catalyzed by T7 RNA polymerase, wherein a region downstream of the promoter region is transcribed from this DNA molecule (transcription template). Hence, a single-stranded RNA molecule transcribed therefrom is an RNA molecule consisting of the following nucleotide sequence R_{candidate0}:
5'-GGUAGAUACGAUGGA
   NNNNNNNNNN NNNNNNNNNN NNNNNNNNNN
   CAUGACGCGCAGCCAA-3'.
When the SELEX method is applied thereto, cDNA molecules (transcription templates) to be used in a next round are prepared as amplification products by use of an RT-PCR method from single-stranded RNA molecules selected in each round. In the step of reverse transcription, a DNA primer complementary to the nucleotide sequence of the 3'-terminal fixed region of the single-stranded RNA molecule, that is, a 3' primer having the following nucleotide sequence:
3' primer:
5'-TTGGCTGCGCGTCATG-3'
   is used to extend, from the 3'-terminus of the primer, a DNA strand complementary to each single-stranded RNA molecule. The single-stranded DNA molecule obtained at this reverse transcription step is a DNA molecule consisting of the following nucleotide sequence:
5'-TTGGCTGCGCGTCATG
   NNNNNNNNNN NNNNNNNNNN NNNNNNNNNN
   TCCATCGTATCTACC-3'.
Then, an upstream PCR primer: 
5'-TGTAATACGACTCACTATA GGTAGATACGATGGA-3'
3'-CCATCTATGCTACCT-N30-
 which contains a nucleotide sequence portion complementary to the 3'-terminal "TCCATCGTATCTACC-3"' portion of the single-stranded DNA molecule, and a downstream PCR primer:
5'-TTGGCTGCGCGTCATG-3'
3'-AACCGACGCGCAGTAC-N₃₀-
which has the same nucleotide sequence as the 5'-terminal "TTGGCTGCGCGTCATG" portion of the single-stranded DNA molecule, are used to perform PCR reaction. Finally, molecules represented by the following nucleotide sequence D_{randumI}:
5'-TGTAATACGACTCACTATA GGTAGATACGATGGA
   NNNNNNNNNN NNNNNNNNNN NNNNNNNNNN
   CATGACGCGCAGCCAA-3'
are prepared as cDNA molecules (transcription templates) to be used in a next round.
The initial "random DNA molecule library" used is a library synthesized by ESPEC CORP. In this library, the random nucleotide sequences thereof are constructed so that the random region of 30 bases, that is, the N₃₀ portion does not comprise, in its sequence, at least the same nucleotide sequences as or nucleotide sequences complementary to the nucleotide sequence "5'-TTGGCTGCGCGTCATG-3'" of the 3' primer (downstream PCR primer) and the partial nucleotide sequence "5'-GGTAGATACGATGGA-3'" contained in "5'-TGTAATACGACTCACTATA GGTAGATACGATGGA-3"' of the upstream PCR primer. That is said, the "range of the random sequence" of the random region of 30 bases, that is, the N₃₀ portion is designed in advance so as to prevent such undesired phenomenon that these primers accidentally hybridize to the random region of 30 bases, that is, the N₃₀ portion when the cDNA molecules (transcription templates) to be used in a next round are prepared by use of the RT-PCR method.

### Preparation of target protein

The GST protein from Schistosoma japonicum, which is utilized as a target protein in the process with use of the SELEX method, is produced as a recombinant protein in transformants that are obtained by transforming host E. coli BL21 strains (In vitro Gene) with a pGEX 6p-2 vector (manufactured by Amersham Biosciences) commercially available as a plasmid vector for recombinant expression containing a gene encoding the GST protein. The pGEX 6p-2 vector has a structure shown in Figure 1 and incorporates therein a drug resistance gene Amp^{r} as a selection marker. The selection of transformants is performed by use of this selection marker. Colonies are formed on a medium supplemented with ampicillin, and thereby transformants carrying the drug resistance gene Amp^{r} are selected. This colony selection is repeated to thereby isolate transformants carrying the pGEX 6p-2 vector of interest.
The obtained transformants are cultured at 37°C. At the point in time when an index of bacterial cell density in the medium: OD₆₀₀ reaches OD₆₀₀ = 0.5, IPTG (final concentration of 0.1 mM) is added into the medium, followed by culture for additional 2 hours. The IPTG added induces the expression of the GST protein-encoding gene from the tac promoter of the pGEX 6p-2 vector, and thereby the recombinantly expressed GST protein is accumulated in the bacterial cells. The cultured bacterial cells are collected, and then the cells are lysed. After that, they are centrifuged (15,000 rpm; 18,800xg) to separate a soluble fraction (cytoplasm component) from an insoluble fraction (membrane component). The supernatant in which the soluble fraction (cytoplasm component) including recombinantly expressed GST protein is dissolved in a PBS buffer solution is collected.

The recombinantly expressed GST protein, which is included in the soluble fraction (cytoplasm component), is isolated by use of an affinity column GSTrap FF targeted for the GST protein. When the supernatant, in which the soluble fraction (cytoplasm component) is dissolved in a PBS buffer solution, is applied to the GSTrap FF column, contaminating proteins other than the recombinantly expressed GST protein are contained in an initial run-through portion. On the other hand, the recombinantly expressed GST protein is temporarily captured by this GSTrap FF column. A protein concentration contained in the PBS buffer solution (pH 7.3) eluted from the column is monitored with absorbance of OD₂₈₀ at a wavelength of 280 nm. At the point in time when the contaminating proteins have been eluted completely, an elution buffer containing 50 mM Tris (pH 8.0) and 10 mM glutathione are poured thereto to elute the recombinantly expressed GST protein. While a protein concentration contained in the elution buffer eluted from the column is monitored with absorbance of OD₂₈₀, protein-containing fractions eluted from the GSTrap FF column under the elution condition are collected. Figure 2 shows a protein concentration (absorbance of OD₂₈₀) peak corresponding to the recombinantly expressed GST protein-containing fractions isolated by the GSTrap FF column purification when a protein concentration (absorbance of OD₂₈₀) contained in the solution eluted from the affinity column is continuously monitored over time.

The eluted fractions collected are applied to a gel filtration column HiLoad 16/10 200 pg (manufactured by Amersham Biosciences). A protein concentration contained in each fraction eluted with a PBS elution buffer solution (pH 7.3) is monitored with absorbance of OD₂₈₀, while a series of fractions corresponding to main protein concentration peak are collected. Figure 3 shows a protein concentration (absorbance of OD₂₈₀) peak corresponding to the recombinantly expressed GST protein-containing fractions when a protein concentration (absorbance of OD₂₈₀) contained in the solution eluted from the gel filtration column is continuously monitored over time. Figure 4 shows a band found in the SDS-PAGE analysis of a protein contained in each of a series of fractions corresponding to the main protein concentration peak, which are collected under the gel filtration conditions. The strength (protein level) of the 26-kDa band corresponding to the GST protein of interest in each lane (each fraction) in the SDS-PAGE analysis result shown in Figure 4 is consistent with the concentration (absorbance of OD₂₈₀) of the protein contained in each fraction shown in Figure 3. A band clearly recognizable other than this 26-kDa band is not found in the molecular weight region. The measurement of the protein concentration (absorbance of OD₂₈₀) contained in each eluted fraction in the column purification is performed by use of an AKTA automatic purification apparatus (manufactured by Amersham Biosciences).
In addition, even a fusion protein consisting of the GST protein and a protein of interest, in which another protein (the protein of interest) is linked to the C-terminus of the GST protein portion serving as an N-terminal fusion partner, is also produced as a recombinant protein. The pGEX 6p-2 vector is originally a vector utilized in the recombinant expression of the fusion protein consisting of the GST protein and the protein of interest, and comprises cloning sites for insertion of a gene encoding the protein of interest, downstream of a coding region of the GST protein. The gene encoding the protein of interest is inserted by use of the cloning sites to construct a vector for expression of the fusion protein consisting of the GST protein and the protein of interest. The fusion protein is produced as a recombinant protein in transformants that are obtained by transforming host E. coli BL21 strains (Invitrogen) with the constructed vector for expression of the fusion protein consisting of the GST protein and the protein of interest.

1 The selection of transformants as well as the production, isolation, and purification of the recombinant protein are performed according to the procedures used for the GST protein.

### Selection of single-stranded RNA molecule exhibiting specific binding affinity to GST protein from Schistosoma japonicum by means of SELEX method

### (1) Preparation of pool of candidate single-stranded RNA molecules (initial RNA pool) used in the first selection round

A group of candidate single-stranded RNA molecules incorporating therein the random nucleotide sequence portion represented by the nucleotide sequence R_{candidate0} is prepared by in vitro transcription using each DNA molecule constituting the "random DNA molecule library" as a transcription template and T7 RNA polymerase (see Fitzwater and Polisky, (1996) Meth. Enz. 267: 275-301). Alcohol-precipitated RNA molecules are separated and collected as a precipitated fraction by centrifugation from a reaction solution containing the group of candidate single-stranded RNA molecules transcribed therefrom. The group of candidate single-stranded RNA molecules collected is re-dissolved in Binding Buffer (50 mM Hepes (pH 7.4), 150 mM NaCl, 5 mM MgCl₂) and prepared into an initial RNA pool having a predetermined RNA molecule concentration. In this step, the RNA molecule concentration in the initial RNA pool is selected at 15 µM.

### (2) Selection of single-stranded RNA molecule exhibiting binding affinity to GST protein by use of Filter separation method

Single-stranded RNA molecules exhibiting binding affinity to the GST protein, which is used as a target protein, are selected according to procedures described below.

A solution comprising the purified recombinant GST proteins and the group of candidate single-stranded RNA molecules contained in the initial RNA pool, which are dissolved in the Binding Buffer, is incubated at room temperature. During the incubation step, single-stranded RNA molecules exhibiting binding affinity to the GST protein form complexes with the GST proteins at a certain ratio in proportion to the level of their binding affinity.

Subsequently, the recombinant GST proteins and the single-stranded RNA molecule/GST protein complexes contained in the incubation solution are separated therefrom by filtration using a filtration manifold (Millipore) and a 13 mmφ nitrocellulose filter. In the group of candidate single-stranded RNA molecules contained in the initial RNA pool, those not forming the single-stranded RNA molecule/GST protein complexes pass through the nitrocellulose filer and are thereby collected into this filtrate.

In the step, the amount of the single-stranded RNA molecules collected into the filtrate is measured and compared with the total amount of the single-stranded RNA molecules added in the incubation solution, and the concentration of the GST proteins formulated in the incubation solution is adjusted so that the ratio determined by this comparison falls within the range of 5% to 50%.

On the other hand, the recombinant GST proteins and the single-stranded RNA molecule/GST protein complexes remaining on the nitrocellulose filter after the separation by filtration are subjected to elution treatment using Binding Buffer containing 6 M urea as an elution buffer. A solution containing the single-stranded RNA molecules eluted from the single-stranded RNA molecule/GST protein complexes on the filter is collected by this elution treatment. The single-stranded RNA molecules contained in the eluted solution collected are subjected to ethanol precipitation treatment, and then separated and collected as a precipitated fraction by centrifugation. A group of thus-separated and collected single-stranded RNA molecules, which are selected in the first round, are subjected to reverse transcription using reverse transcriptase from avian myeloblastosis virus (Roche Diagnostics) to prepare cDNA therefrom.

All the RNA molecules in the group of single-stranded RNA molecules, which are selected in the first round, carry the nucleotide sequence "CAUGACGCGCAGCCAA-3"' as their 3'-terminal fixed regions. Thus, a 3' primer having a nucleotide sequence "5'-TTGGCTGCGCGTCATG-3'" complementary to the nucleotide sequence of the 3'-terminal fixed region is used in the reverse transcription to extend, from the 3'-terminus of the primer, a DNA strand having a nucleotide sequence complementary to the "random region of 30 bases" and the 5'-terminal fixed region "5'-GGUAGAUACGAUGGA" of each single-stranded RNA molecule.

The cDNA obtained at the step of reverse transcription is further used as a template to perform total 15 cycles of PCR reaction using the following PCR primer pair:
a 3' primer (downstream PCR primer): 5'-TTGGCTGCGCGTCATG-3';
an upstream PCR primer: 5'-TGTAATACGACTCACTATA GGTAGATACGATGGA-3',
with a DNA polymerase enzyme Ex-Taq (Takara Bio). The obtained PCR amplification products have, at each of the terminals, fixed regions from the 3' primer (downstream PCR primer) and from the upstream PCR primer, respectively and have, between these fixed regions, the "selected nucleotide sequence region of 30 bases" which is available in the transcription of the "random region of 30 bases" in each of the group of single-stranded RNA molecules selected in the first round.

The obtained PCR amplification products are separated and collected as double-stranded DNA molecules by hybridization between the complementary strands contained in this PCR reaction solution. A group of thus-separated and collected double-stranded DNA molecules (the first round screening DNA molecule group) is used as transcription templates in the preparation of a pool of candidate single-stranded RNA molecules (RNA pool) utilized in a next selection round.
The "random DNA molecule library" utilized in the preparation of the initial RNA pool is designed so that the content of each kind of transcription template (double-stranded DNA molecule) is the identical proportion in principle to each other. However, in "the first round screening DNA molecule group" utilized in the preparation of the RNA pool for the 2nd selection round, the content of each kind of transcription template (double-stranded DNA molecule) from the single-stranded RNA molecule exhibiting binding affinity to the GST protein corresponds to the level of the binding affinity that the singles-stranded RNA molecule has. Specifically, the transcription templates (double-stranded DNA molecules) utilized in the transcription of the single-stranded RNA molecules exhibiting a high binding affinity to the GST protein are "concentrated" in "the first round screening DNA molecule group".
The preparation of the RNA pool for the 2nd selection round is performed by in vitro transcription using T7 RNA polymerase from each transcription template (double-stranded DNA molecule) constituting "the first screening DNA molecule group" according to the procedures described in the step of (1) Preparation of initial RNA pool. The obtained RNA molecules are redissolved in Binding Buffer according to the same procedures of separation, collection, and redissolution as those mentioned above, and prepared into a 2nd RNA pool having a predetermined RNA molecule concentration. In this step, the concentration of the RNA molecules contained in the 2nd RNA pool is selected at 10 µM.

The 2nd selection round is also performed according to the same procedures as the first selection round. In this round, the amount of the single-stranded RNA molecules collected into the filtrate is measured and compared with the total amount of the single-stranded RNA molecules added in the incubation solution, and the concentration of the GST proteins blended in the incubation solution is adjusted so that the ratio determined by this comparison falls within the range of 5% to 50%. Specifically, in the RNA pool for the 2nd selection round, the content of the single-stranded RNA molecules exhibiting binding affinity to the GST protein is increased. In response to this increase, the concentration of the GST proteins blended in the incubation solution is decreased in the second selection round as compared with that used in the first selection round. A "2nd round screening DNA molecule group" is prepared in similar manner to the first round by reverse transcription and PCR reaction from the single-stranded RNA molecules exhibiting binding affinity to the GST protein, which are selected in this 2nd selection round. The transcription templates (double-stranded DNA molecules) utilized in the transcription of the single-stranded RNA molecules exhibiting high binding affinity to the GST protein are "concentrated" in "the 2nd round screening DNA molecule group" as compared with "the first round screening DNA molecule group".

Subsequently, the number of screening rounds is increased in order. At the point in time when the total number of rounds has reached 13, that is, at the point in time when the first to 13th rounds have been completed, " the 13th round screening DNA molecule group" is collected as a transcription template (double-stranded DNA molecule) group selected by the SELEX method using the Filter separation method and utilized for the transcription of single-stranded RNA molecules exhibiting higher binding affinity.

To keep the "concentrated" ratio in the desired range in each round of selection, the concentration of the GST protein blended in the incubation solution is decreased with increases in the number of rounds, as described above. However, the total concentration of the single-stranded RNA molecules is gradually decreased with increases in the number of rounds to prevent the ratio of the total concentration of the single-stranded RNA molecules to the concentration of the GST protein from becoming exceedingly large. Specifically, in the first selection round through 13th selection round, the concentration of the GST protein blended in the incubation solution is decreased from 15 µM in the first selection round to 1 nM in the 13th selection round. On the other hand, the total concentration of the single-stranded RNA molecules is decreased from 15 µM in the first selection round to 100 nM in the 13th selection round. Thus, the ratio of the total concentration of the single-stranded RNA molecules to the concentration of the GST proteins in the incubation solution is 1:1 in the first selection round and 100:1 in the 13th selection round and ranges from 1:1 to 100:1 in the rounds between them.

### (3) Selection of single-stranded RNA molecule exhibiting binding affinity to GST protein by use of surface plasmon resonance biosensor

At the point in time when the 13th selection round has been completed, an additional decrease in the concentration of the GST protein blended in the incubation solution is difficult in keeping the "concentrated" ratio required in screening by the SELEX method using the Filter separation method. Therefore, the selection shifts to screening by the SELEX method using a surface plasmon resonance biosensor.

The surface plasmon resonance detection apparatus used is a BIAcore model 2000 (manufactured by Biacore). The surface of a CM4 BIACORE chip (Biacore) for the surface plasmon resonance detection apparatus is subjected to EDC-NHS activation treatment by use of a Biacore amine coupling kit. The GST protein is immobilized by use of the amine coupling agent onto this chip surface that has undergone the EDC-NHS activation treatment.

On the other hand, the preparation of an RNA pool utilized in selection is performed by in vitro transcription using T7 RNA polymerase from each transcription template (double-stranded DNA molecule) constituting " the 13th screening DNA molecule group" according to the procedures described in the step of (1) Preparation of initial RNA pool. The obtained RNA molecules are redissolved in Binding Buffer according to the same procedures of separation, collection, and redissolution as above and prepared into a 14th RNA pool having a predetermined RNA molecule concentration. In this step, the concentration of the RNA molecules in the 14th RNA pool utilized in screening by the SELEX using the surface plasmon resonance biosensor is selected at 100 nM.

The surface plasmon resonance detection apparatus comprises four flow cells on the chip, on the surface of which chip the GST protein is immobilized. Into each of these flow cells, the solution of the 14th RNA pool is poured at a flow rate of 30 µL/min. to allow the single-stranded RNA molecules to bind to the GST protein immobilized on the chip. At the point in time when the amount of the solution of the 14th RNA pool injected into each flow cell has reached 100 µL, the solution is changed to Binding Buffer, which is in turn poured thereinto at a flow rate of 30 µL/min. to dissociate the single-stranded RNA molecules bound with the GST protein and elute the dissociated single-stranded RNA molecules. The eluted solution containing the single-stranded RNA molecules that have undergone the temporary binding with the GST protein and the subsequent dissociation and elution is separated into fractions of 300 µL each (for 10 minutes each), and five fractions are collected therefrom. Of them, four fractions except for the initial one fraction are subjected, in similar manner to the first round, to reverse transcription and PCR reaction to prepare "the 14th round screening DNA molecule group".
The preparation of a 15th RNA pool is performed by in vitro transcription using T7 RNA polymerase from each transcription template (double-stranded DNA molecule) constituting " the 14^{th} round screening DNA molecule group" according to the procedures described in the step of (1) Preparation of initial RNA pool. The obtained RNA molecules are redissolved in Binding Buffer according to the same procedures of separation, collection, and redissolution as above and prepared into a 15th RNA pool having a predetermined RNA molecule concentration. In this step, the concentration of the RNA molecules contained in the 15th RNA pool is selected at 100 nM.

Screening by the SELEX method using the surface plasmon resonance biosensor is performed by use of the 15th RNA pool according to the similar procedures to those for the 14th round. Specifically, the final fraction containing the single-stranded RNA molecules that are finally eluted by use of an eluent is collected and combined with the eluted solution containing the single-stranded RNA molecules that have undergone the temporary binding with the GST protein immobilized on the chip and the subsequent dissociation and elution. "The 15th round screening DNA molecule group" is prepared in similar manner by reverse transcription and PCR reaction from the single-stranded RNA molecules exhibiting binding affinity to the GST protein, which are selected in the 15th selection round.

### (4) Selection of single-stranded RNA molecule exhibiting binding affiniy to GST protein by use of GST protein bound on GST affinity beads

Further selection is performed by use of the GST protein immobilized on commercially available GST affinity beads (Amersham Biosciences) via binding with a substrate glutathione.

The preparation of an RNA pool utilized in selection is performed by in vitro transcription using T7 RNA polymerase from each transcription template (double-stranded DNA molecule) constituting " the 15th round screening DNA molecule group" according to the procedures described in the step of (1) Preparation of initial RNA pool. The obtained RNA molecules are redissolved in Binding Buffer according to the same procedures of separation, collection, and redissolution as above and prepared into a 16th RNA pool having a predetermined RNA molecule concentration. In this step, the concentration of the RNA molecules in the 16th RNA pool utilized in screening by the SELEX using the GST affinity beads is selected at 100 nM.

On the other hand, the GST affinity beads are dipped in a solution containing the GST protein at the protein concentration of 1 nM to thereby immobilize the GST protein onto the GST affinity beads via binding with the substrate glutathione. The GST affinity beads are washed with Binding Buffer to remove un-immobilized GST protein therefrom.

The 16th RNA pool is added to the GST affinity beads bound with the GST protein. In the mixture solution having an initial RNA molecule concentration of 100 nM, the single-stranded RNA molecules are allowed to further bind to the GST proteins bound on the GST affinity beads. Specifically, the single-stranded RNA molecules having binding affinity to the GST protein are picked out, regardless of the presence or absence of the binding of glutathione to the GST protein.

Then, the liquid phase containing the single-stranded RNA molecules is removed by filtration. The GST affinity beads are washed with Binding Buffer to remove the residual solution containing the single-stranded RNA molecules. After this rinsing treatment, the GST affinity beads are dipped in Binding Buffer containing glutathione (at concentration of 10 mM) to thereby elute the GST proteins bound on the GST affinity beads. Subsequently, the single-stranded RNA molecules bound with the eluted GST proteins are dissociated from the GST proteins. The dissociated single-stranded RNA molecules are subjected to ethanol precipitation treatment and separated and collected as a precipitated fraction by centrifugation. "The 16th round screening DNA molecule group" is prepared by reverse transcription and PCR reaction according to the same procedures as above from the single-stranded RNA molecules exhibiting binding affinity to the GST protein, which are selected in this 16th selection round.

The preparation of a 17th RNA pool is performed by in vitro transcription using T7 RNA polymerase from each transcription template (double-stranded DNA molecule) constituting " the 16th round screening DNA molecule group" according to the procedures described in the step of (1) Preparation of initial RNA pool. The obtained RNA molecules are redissolved in Binding Buffer according to the same procedures of separation, collection, and redissolution as above and prepared into a 17th RNA pool having a predetermined RNA molecule concentration. In this step, the concentration of the RNA molecules contained in the 17th RNA pool is selected at 100 nM.

Screening by the SELEX method using the GST proteins bound on the GST affinity beads is performed by use of this 17th RNA pool according to the same procedures as above. Specifically, the single-stranded RNA molecules that have undergone the temporary binding with the GST proteins immobilized on the GST affinity beads and the subsequent dissociation from the GST proteins after the elution of the GST protein are subjected to ethanol precipitation treatment and separated and collected as a precipitated fraction by centrifugation. "The 17th round screening DNA molecule group" is prepared by reverse transcription and PCR reaction according to the same procedures as above from the single-stranded RNA molecules exhibiting binding affinity to the GST protein, which are selected in this 17th selection round.

### Filter Binding Assay

Subsequently, the preparation of an 18th RNA pool is performed by in vitro transcription using T7 RNA polymerase from each transcription template (double-stranded DNA molecule) constituting " the 17th round screening DNA molecule group" according to the procedures described in the step of (1) Preparation of initial RNA pool. The obtained RNA molecules are redissolved in Binding Buffer according to the same procedures fo separation, collection, and redissolution as above and prepared into an 18th RNA pool having a predetermined RNA molecule concentration. In this step, the concentration of the RNA molecules contained in the 18th RNA pool is selected at 40 nM. Of substrate NTPs utilized in the transcription reaction, ATP used is α-³²P ATP (Amersham Biosciences), with which the single-stranded RNA molecules are radio-labeled.

It is sufficiently expected from the results of a series of the screening processes that a group of single-stranded RNA molecules contained in this 18th RNA pool exhibits high and specific binding affinity to the GST protein. To predict the level of their equilibrium dissociation constants, Filter Binding Assay is performed according to procedures described below.

In an incubation solution containing the radio-labeled 18th RNA pool and the GST protein, the concentration of the group of single-stranded RNA molecules is set to 20 nM, whereas the concentration of the GST protein is selected at 2-fold dilution series concentrations of 1000 nM to 62.5 nM. The solution is incubated at room temperature for 10 minutes. Next, 100 µL of each incubation solution is spotted onto a nitrocellulose filter. The GST protein and single-stranded RNA molecule/GST protein complexes are adsorbed onto the filter. Then, the filter is washed with Binding Buffer to remove single-stranded RNA molecules not forming the complexes.

The amount of γ-rays emitted from the radio-label is measured with an imaging plate and a bio-imaging analyzer BAS2500 manufactured by Fujifilm. The imaging plate is exposed by the γ-rays emitted in each spot on the filter, and the amount of exposure thereof is measured on BAS2500. Specifically, the mount of exposure is visualized with ImageReader (Fujifilm) and converted into numbers with ImageGauge ver.4.0 (Fujifilm).

Figure 5 shows one example of a measurement result of Filter Binding Assay. The group of single-stranded RNA molecules contained in the 18th RNA pool was shown by calculation to exhibit K_{D} = approximately 400 nM as an apparent equilibrium dissociation constant.

### Cloning

The transcription template (double-stranded DNA molecule) group contained in "the 17th round screening DNA molecule group" picked out by a series of the processes in the SELEX method is cloned by use of a commercially available TA cloning kit (Invitrogen). Specifically, the RT-PCR amplification product (double-stranded DNA molecule) group is ligated to cloning vectors pCR2.1 according to the standard protocols included in the kit. After this ligation, E. coli TOP10 strain is transformed with the cloning vectors.

Subsequently, transformants carrying the cloning vectors are subjected to colony screening using a selection marker from the cloning vector according to a conventional method.
Purification of vector and Determination of nucleotide sequence of inserted DNA fragment
Total 40 clones are picked up at random from the transformants that have been selected by colony screening. Then, an analysis is made on the nucleotide sequences of the DNA fragments inserted in the cloning vectors thereof.

Each of the selected clones is additionally cultured. The plasmid vector carried by each of the clones is separated and purified by use of a QIAprep Spin Miniprep kit (QIAGEN). An analysis is made on the nucleotide sequence of the "selected nucleotide sequence region of 30 bases" unique to each clone located between the 5'-terminal fixed region from the upstream PCR primer and the 3'-terminal fixed region from the downstream PCR primer in the DNA fragment portion inserted in this purified plasmid vector carried by each clone. Specifically, a sequencing primer:
5'-GGTAGATACGATGGA-3'
which is corresponding to "GGTAGATACGATGGA" of the 5'-terminal fixed region from the upstream PCR primer in the inserted DNA fragment portion is used to perform nucleic acid strand extension reaction for sequencing using a BigDye kit (Applied Biosystems) and the inserted DNA fragment portion as a template. The product for sequencing thus prepared is analyzed with an ABI model 310 sequencer to make an analysis on the nucleotide sequences of the "selected nucleotide sequence region of 30 bases" unique to each clone and the 3'-terminal fixed region from the downstream PCR primer.
When the nucleotides sequence of the "selected nucleotide sequence region of 30 bases" was compared among the total 40 clones, it was confirmed that these 40 clones include many clones having the same nucleotide sequences. Specifically, the nucleotide sequence of the "selected nucleotide sequence region of 30 bases" unique to each clone was divided into only 3 types among the total 40 clones. These 3 types of nucleotide sequences found are the partial nucleotide sequences of the DNA fragments inserted in No. 3, No. 5, and No. 26 clones described below when indicated as the nucleotide sequences of the region "GGTAGATACGATGGA" of 5'-terminal fixed region from the upstream PCR primer used as a sequencing primer, the "selected nucleotide sequence region of 30 bases", and the 3'-terminal fixed region from the downstream PCR primer.

The partial nucleotide sequence of the DNA fragment inserted in No. 3 clone: 
GGTAGATACGATGGA TGGTTGTGTAAAGGTGGTCGTATCCGCCGA
CATGACGCGCAGCCAA
 the partial nucleotide sequence of the DNA fragment inserted in No. 5 clone: 
GGTAGATACGATGGA CTAACTGCGCAAATTACTCGTATTAGCCGA
CATGACGCGCAGCCAA; and
 the partial nucleotide sequence of the DNA fragment inserted in No. 26 clone: 
GGTAGATACGATGGA TACCGAAAAATTAGTGTCGTTGACTGCAA
CATGACGCGCAGCCAA.
 Thus, the group of the transcription templates (double-stranded DNA molecules) contained in the "17th screening DNA molecule group" contain, as the corresponding transcription templates (double-stranded DNA molecules), transcription templates (double-stranded DNA molecules) represented by the nucleotide sequences of No. 3, No. 5, and No. 26 clones described below comprising the 5'-terminal fixed region "5'-TGTAATACGACTCACTATA GGTAGATACGATGGA-3"' from the upstream PCR primer.

The nucleotide sequence of the DNA fragment inserted in No. 3 clone: 
TGTAATACGACTCACTATA GGTAGATACGATGGA
TGGTTGTGTAAAGGTGGTCGTATCCGCCGA CATGACGCGCAGCCAA
 the nucleotide sequence of the DNA fragment inserted in No. 5 clone: 
TGTAATACGACTCACTATA GGTAGATACGATGGA
CTAACTGCGCAAATTACTCGTATTAGCCGA CATGACGCGCAGCCAA;
 and
the nucleotide sequence of the DNA fragment inserted in No. 26 clone: 
TGTAATACGACTCACTATA GGTAGATACGATGGA
TACCGAAAAATTAGTGTCGTTGACTGCAA CATGACGCGCAGCCAA.
 The plasmids carried in these 3 types of clones, No. 3, No. 5, and No. 26 clones, have been deposited domestically since Mar. 7, 2005 as deposition Nos. FERM P-20439 (designated as "pCR-GST No. 3" as indication for identification), FERM P-20440 (designated as "pCR-GST No. 5"), and FERM P-20441 (designated as "pCR-GST No. 26"), respectively, with International Patent Organism Depositary, National Institute of Advanced Industrial Science and Technology (Tsukuba Central 6, 1-1 Higashi, Tsukuba, Ibaraki 305-8566, Japan).
The fusion protein consisting of the GST protein and the protein of interest was used instead of the GST protein to select a group of single-stranded RNA molecules exhibiting binding affinity to the fusion protein according to the same procedures as in the step of "Selection of single-stranded RNA molecule exhibiting binding affinity to GST protein by use of Filter separation method". It was confirmed that the group of single-stranded RNA molecules picked out contains those having the same nucleotide sequences as single-stranded RNA molecules transcribed from the transcription templates (double-stranded DNA molecules) cloned in the 3 types of clones, No. 3, No. 5, and No. 26 clones. Specifically, the single-stranded RNA molecules transcribed from the transcription templates (double-stranded DNA molecules) cloned in the 3 types of clones, No. 3, No. 5, and No. 26 clones are confirmed to exhibit binding affinity to the GST protein portion serving as an N-terminal fusion partner in the fusion protein consisting of the GST protein and the protein of interest.

### Determination of equilibrium dissociation constant

The equilibrium dissociation constants of single-stranded RNA molecule/GST protein complexes are evaluated as an indicator of the binding affinity to the GST protein exhibited by the single-stranded RNA molecules transcribed from the transcription templates (double-stranded DNA molecules) cloned in the 3 types of clones, No. 3, No. 5, and No. 26 clones.
The plasmid vectors "pCR-GST No. 3", "pCR-GST No. 5", and "pCR-GST No. 26" respectively carried by the clones are separately used as a template to perform PCR reaction using
a downstream primer (3' primer):
5'-TTGGCTGCGCGTCATG-3' and
an upstream primer:
5'-TGTAATACGACTCACTATA An GGTAGATACGATGGA-3'
comprising polyA (An) added upstream of a 5' primer "GGTAGATACGATGGA" and a "T7 promoter region (TGTAATACGACTCACTATA)" functioning as a promoter region in transcription catalyzed by T7 RNA polymerase, which is located on the 5'-terminal side of the polyA. The obtained PCR products comprise the "T7 promoter region", "polyA (An) portion", and "5' primer portion" from the the upstream primer as the 5'-terminal fixed region at the 5'-terminal side and the "3' primer portion" from the downstream primer as the 3'-terminal fixed region at the 3'-terminal side, respectively.

The PCR products are purified, and the purified PCR products are then used as transcription templates to prepare single-stranded RNA molecules by in vitro transcription reaction using T7 RNA polymerase according to the transcription conditions described above. The transcribed single-stranded RNA molecules are such molecules in which polyA (An) is added to the 5'-terminal side of the single-stranded RNA molecule (RNA adaptor molecule) exhibiting high binding affinity to the GST protein.

Synthetic biotinylated polydT (Hokkaido System Science) is allowed to bind to the surface of an SA BIACORE chip (Biacore). The single-stranded RNA molecules (RNA adaptor molecules) comprising polyA (Aₙ) added on the 5'-terminal side are annealed with the synthetic biotinylated polydT immobilized on the chip surface. As a result, the single-stranded RNA molecules (RNA adaptor molecules) are immobilized thereon via the hybrid bonding between the polydT and the polyA (An).

This chip on which the single-stranded RNA molecules (RNA adaptor molecules) are immobilized is utilized to measure the process of formation of complexes of the single-stranded RNA molecules (RNA adaptor molecules) and GST proteins and the process of dissociation thereof by use of a surface plasmon resonance detection apparatus. The results are analyzed to determine the equilibrium dissociation constants of the complexes.

In the surface plasmon resonance detection apparatus BIAcore model 2000, buffer solutions containing the GST protein at variety of concentrations are poured at a flow rate of 30 µL/min. into the flow channel of the chip to form complexes of the single-stranded RNA molecules (RNA adaptor molecules) immobilized on the chip and the GST protein. Time-dependent changes in signal intensity associated with increases in the amount of the complexes in this process of formation (binding process) of the complexes are observed for 2 minutes. Subsequently, the buffer solutions are changed to buffer solutions free from the GST protein, which are also poured at a flow rate of 30 µL/min. to dissociate the formed complexes. Time-dependent changes in signal intensity associated with decreases in the amount of the complexes in this dissociation process are observed for 3 minutes.

Parameter fitting is conducted by use of BIA Evolution ver. 3.1 for the concentrations of the GST protein in the poured GST protein solutions and the time-dependent changes in signal intensity measured in this process, to thereby determine equilibrium dissociation constant K_{D}.

Figures 6, 7, and 8 show results of measuring single-stranded RNA molecules (RNA adaptor molecules) from the DNA fragments inserted in the 3 types of clones, No. 3, No. 5, and No. 26 clones, respectively, for the process of formation of their complexes with the GST protein and the process of dissociation of the complexes.

### Industrial Applicability

The RNA aptamer molecule according to the present invention exhibits specific binding affinity to the GST protein from Schistosoma japonicum and can therefore be utilized as a nucleic acid ligand substrate having specific binding affinity to the GST protein in the affinity column purification of the fusion protein comprising the GST protein as an N-terminal fusion partner or as a nucleic acid ligand substrate having specific binding affinity to the GST protein in the preparation of a labeling substance intended for detection of the fusion protein comprising the GST protein as an N-terminal fusion partner.

## Claims

1. An RNA aptamer molecule capable of binding to a GST protein from Schistosoma japonicum, **characterized in that**
the RNA aptamer molecule is composed of
single-stranded RNA having the following nucleotide sequence I (SEQ ID NO: 1):

GGUAGAUACGAUGGA UGGUUGUGUAAAGGUGGUCGUAUCCGCCGA
CAUGACGCGCAGCCAA 61.

2. An RNA aptamer molecule capable of binding to a GST protein from Schistosoma japonicum, **characterized in that**
the RNA aptamer molecule is composed of
single-stranded RNA having the following nucleotide sequence II (SEQ ID NO: 2):

GGUAGAUACGAUGGA CUAACUGCGCAAAUUACUCGUAUUAGCCGA
CAUGACGCGCAGCCAA 61.

3. An RNA aptamer molecule capable of binding to a GST protein from Schistosoma japonicum, **characterized in that**
the RNA aptamer molecule is composed of
single-stranded RNA having the following nucleotide sequence III (SEQ ID NO: 3):

GGUAGAUACGAUGGA UACCGAAAAAUUAGUGUCGUUGACUGCAA
CAUGACGCGCAGCCAA 60.

4. Use of an RNA aptamer molecule as claimed in any one of claims 1 to 3, **characterized in that**
the RNA aptamer molecule is used as a nucleic acid ligand substrate having specific binding affinity to a GST protein from Schistosoma japonicum, in preparation of an affinity column intended for affinity column purification of the GST protein or a fusion protein comprising the GST protein as a fusion partner and another protein linked to the C-terminus of the GST protein.

5. Use of an RNA aptamer molecule as claimed in to any one of claims 1 to 3, **characterized in that**
the RNA aptamer molecule is used as a nucleic acid ligand substrate having specific binding affinity to a GST protein from Schistosoma japonicum, in preparation of a labeling substance intended for detection of a fusion protein comprising the GST protein as a fusion partner and another protein linked to the C-terminus of the GST protein.
